(19) Europäisches Patentamt
European Patent Office
Office européen des brevets

(11) EP 2 589 337 A1

(12) **EUROPÄISCHE PATENTANMELDUNG**

(43) Veröffentlichungstag:
08.05.2013 Patentblatt 2013/19

(51) Int Cl.:
*A61B 5/145* (2006.01) *G01N 33/487* (2006.01)

(21) Anmeldenummer: 11187840.1

(22) Anmeldetag: 04.11.2011

(84) Benannte Vertragsstaaten:
AL AT BE BG CH CY CZ DE DK EE ES FI FR GB GR HR HU IE IS IT LI LT LU LV MC MK MT NL NO PL PT RO RS SE SI SK SM TR
Benannte Erstreckungsstaaten:
BA ME

(71) Anmelder:
• Roche Diagnostics GmbH
68305 Mannheim (DE)
Benannte Vertragsstaaten:
DE
• F. Hoffmann-La Roche AG
4070 Basel (CH)
Benannte Vertragsstaaten:
AL AT BE BG CH CY CZ DK EE ES FI FR GB GR HR HU IE IS IT LI LT LU LV MC MK MT NL NO PL PT RO RS SE SI SK SM TR

(72) Erfinder:
• Chang, Tso-Yu
New Taipei City 238 (TW)
• Wang, Peng-Kun
Xiangshan Dist.
Hsinchu City 300 (TW)

(74) Vertreter: Pfiz, Thomas et al
Patentanwälte Wolf & Lutz
Hauptmannsreute 93
70193 Stuttgart (DE)

(54) **Analytisches Handgerät und Verfahren zu dessen Betrieb**

(57) In einem analytischen Handgerät, insbesondere für Blutzuckertests, mit einer austauschbaren Testbandkassette (18), die ein mit einer Vielzahl von analytischen Hilfsmitteln (22) versehenes Testband (20) enthält, einem einen Gleichstrommotor (30) und ein mit der Testbandkassette (18) koppelbares Getriebe (34,36) aufweisenden Bandantrieb (14) zum sukzessiven Bereitstellen der analytischen Hilfsmittel (22), und einer Regeleinrichtung (16) zur Drehzahlregelung des Gleichstrommotors (30), wird vorgeschlagen, dass die Regeleinrichtung (16) einen an dem Gleichstrommotor (30) angeordneten Drehgeber (38) zur Erfassung der IST-Drehzahl der Motorabtriebswelle (32) aufweist.

Fig.1

EP 2 589 337 A1

**Beschreibung**

Die Erfindung betrifft ein analytisches Handgerät, insbesondere für Blutzuckertests, mit einer austauschbaren Testbandkassette, die ein mit einer Vielzahl von analytischen Hilfsmitteln versehenes Testband enthält, einem einen Gleichstrommotor und ein mit der Testbandkassette koppelbares Getriebe aufweisenden Bandantrieb zum sukzessiven Bereitstellen der analytischen Hilfsmittel, und einer Regeleinrichtung zur Drehzahlregelung des Gleichstrommotors. Die Erfindung betrifft weiterhin ein Verfahren zum Betrieb eines solchen analytischen bzw. medizinischen Handgeräts.

Derartige Testbandsysteme wurden bereits in einer Reihe von Patentanmeldungen von den Anmeldern vorgeschlagen, um gegenüber den am Markt befindlichen Streifensystemen weitere Anwendervorteile zu gewinnen. Für die Praxis muss neben einer zuverlässigen Positionierung der Testelemente auch gewährleistet sein, dass eine vorgegebene Bereitstellungszeit für die Einzeltests mit möglichst geringem Aufwand eingehalten werden kann.

Ausgehend hiervon liegt der Erfindung die Aufgabe zugrunde, die im Stand der Technik vorgeschlagenen Systeme und Verfahren weiter zu verbessern und eine rasche Testelementbereitstellung bei vertretbarer Bandbelastung mit einfachen Mitteln zu erreichen.

Zur Lösung dieser Aufgabe wird die im Patentanspruch 1 bzw. 14 angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, einen hochdrehenden Motor durch eine möglichst einfache Drehzahlregelung zu ergänzen. Dementsprechend wird erfindungsgemäß vorgeschlagen, dass die Regeleinrichtung einen an dem Gleichstrommotor angeordneten Drehgeber zur Erfassung der IST-Drehzahl der Motorabtriebswelle aufweist. Damit ist es möglich, kostengünstige kompakte Kleinmotoren einzusetzen und deren bauartbedingte Drehzahlvarianz zu kompensieren, ohne dass bereits bei der Herstellung eine Motorkalibrierung erforderlich wäre. Gleichzeitig kann die Motordrehzahl verbrauchsabhängig dahingehend kontrolliert werden, dass eine gleichbleibende Testbandgeschwindigkeit und damit eine konstante Testbereitstellungszeit erreicht wird. Die direkte Erfassung der Drehung der Motorabtriebswelle ermöglicht die erforderliche Genauigkeit der Regelung mit kurzer Totzeit, wobei die Erkenntnis zugrunde gelegt wird, dass durch die dem Motor nachgeordnete Antriebskopplung des Testbandes keine nennenswerten Störeinflüsse auftreten.

Um mit möglichst einfachen Mitteln eine hochpräzise Drehwinkelerfassung zu ermöglichen, ist es vorteilhaft, wenn der Drehgeber einen die Drehung der Motorabtriebswelle optisch abtastenden und dabei eine zur Drehzahl proportionale Rate von elektrischen Impulsen als Ausgangssignal erzeugenden opto-elektronischen Encoder aufweist.

Eine weitere Verbesserung lässt sich dadurch erzielen, dass der Drehgeber einen drehfest auf der Motorabtriebswelle sitzenden Unterbrecher, insbesondere ein Flügelrad oder eine Lochscheibe und eine mit dem Unterbrecher zusammenwirkende gerätefeste Lichtschranke, vorzugsweise eine Gabellichtschranke aufweist.

Zur möglichst präzisen Signalauswertung ist vorteilhafterweise ein zur Erzeugung von Zeittaktimpulsen ausgebildeter Taktgeber und ein Zähler zum Zählen von Zeittaktimpulsen zwischen zwei Signalflanken eines Ausgangssignals des Drehgebers vorgesehen.

Für eine möglichst variable Regelung ist es günstig, wenn die Regeleinrichtung einen Vergleicher zur Bildung einer Regeldifferenz zwischen der IST-Drehzahl und einer als Führungsgröße vorgegebenen SOLL-Drehzahl aufweist. Hierbei ist es auch von Vorteil, wenn die Regeleinrichtung einen eingangsseitig mit einer Regeldifferenz beaufschlagbaren Regelprozessor zur Einstellung einer Soll-Drehzahl in einem geschlossenen Regelkreis aufweist.

Für eine möglichst rasche Verringerung einer Regelabweichung sollte der Regelprozessor vorzugsweise durch eine Software-Routine gebildete Proportional- und Integral-Regelglieder aufweisen.

Zur genauen Stellgrößeneinstellung ist es vorteilhaft, wenn die Regeleinrichtung einen Pulsweitenmodular als Stellglied zur Ansteuerung des Gleichstrommotors mit einer pulsweitenmodulierten Gleichspannung aufweist.

Vorteilhafterweise ist die Regeleinrichtung dazu eingerichtet, eine Bandgeschwindigkeit des Testbandes von 15 +/- 2 mm/s mit einer Einstellzeit im Bereich von 0,1 bis 0,25 s zu erreichen.

Ein weiterer Gebrauchsvorteil für den Benutzer lässt sich dadurch erreichen, dass die Regeleinrichtung einen Sollwertgenerator zur Festlegung einer aktuellen SOLL-Drehzahl nach Maßgabe einer gleichbleibenden Bereitstellungszeit für die jeweilige Bereitstellung der analytischen Hilfsmittel aufweist.

In diesem Zusammenhang ist es auch vorteilhaft, einen Sollwertspeicher zur Speicherung einer Sollwerttabelle vorzusehen, in welcher einer Testnummer der fortlaufend nummerierten analytischen Hilfsmittel jeweils ein Wert für die SOLL-Drehzahl des Gleichstrommotors zugeordnet ist.

Um auch bei einem Kassettenwechsel eine Verbrauchsinformation bereitstellen zu können, ist es vorteilhaft, wenn die Testbandkassette mit einem insbesondere als RFID-Chip ausgebildeten Speichermittel zur verbrauchsabhängigen Speicherung einer Testnummer des aktuell bereitzustellenden analytischen Hilfsmittels versehen ist.

Der Testbandantrieb ist vorteilhafterweise dazu eingerichtet, die analytischen Hilfsmittel bei Bedarf durch Bandtransport von einer gegenüber der Umgebung abgeschirmten Vorratsspule abzuziehen und an einer Applikationsstelle eines Gerätegehäuses bereitzustellen.

In verfahrensmäßiger Hinsicht wird die eingangs genannte Aufgabe dadurch gelöst, dass die IST-Drehzahl der Motorabtriebswelle durch einen an dem Gleichstrommotor angeordneten Drehgeber der Regeleinrichtung erfasst wird.

Im Folgenden wird die Erfindung anhand eines in der Zeichnung dargestellten Ausführungsbeispiels näher erläutert. Es zeigen:

Fig. 1     ein analytisches Handgerät zum Einsatz einer wechselbaren Testbandkassette in einer per- spektivischen Darstellung mit Detailvergrößerung eines Drehgebers;

Fig. 2     ein Blockschaltbild einer Regeleinrichtung für den Bandantrieb;

Fig. 3     ein Zeitdiagramm der Ausgangssignale des Drehgebers über den Taktimpulsen eines Zeittaktgebers.

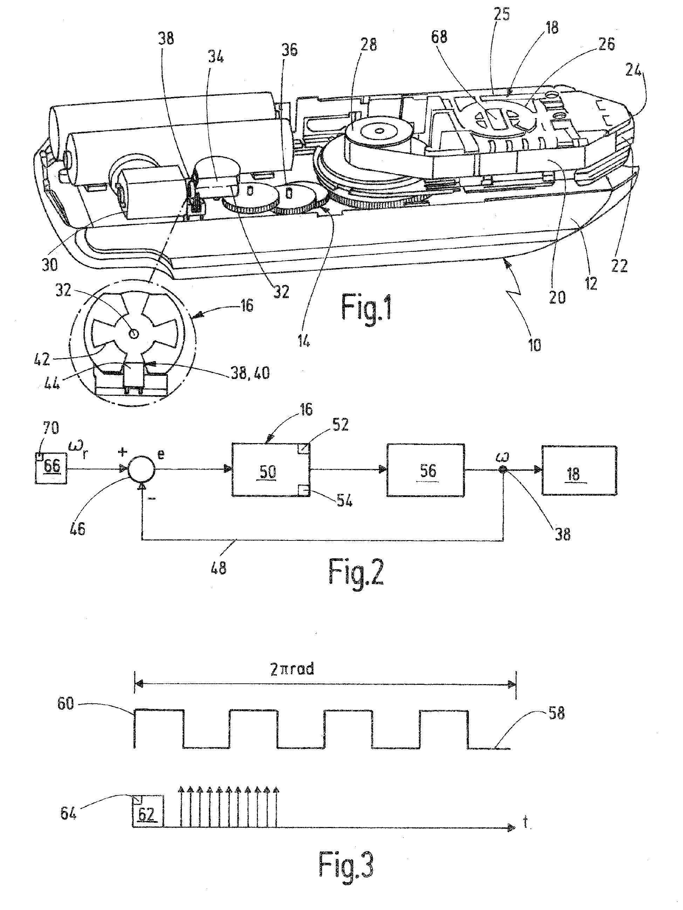

Das in Fig. 1 dargestellte Handgerät lässt sich von einem Anwender in der Hand tragen und dabei für rasche Blutzuckermessungen vor Ort einsetzen. Das Mobilgerät 10 umfasst zu diesem Zweck ein ohne Abdeckung gezeigtes Gehäuse 12 mit einem Bandantrieb 14 und einer darauf einwirkenden Regeleinrichtung 16 zur Bandtransportkontrolle einer als Verbrauchsmittel einwechselbaren Testbandkassette 18.

Die ohne Deckel dargestellte Testbandkassette 18 enthält ein Testband 20, das abschnittsweise mit trockenchemischen Testfeldern 22 versehen ist, die im Bereich einer Umlenkspitze als Applikationsstelle 24 an ihrer freien Vorderseite mit Blut bzw. Körperflüssigkeit beaufschlagbar sind. Zugleich kann dort eine rückseitige Vermessung des momentan bzw. aktuell benutzten Testfelds 22 durch eine nicht gezeigte photometrische Messeinheit erfolgen. Das Testband 16 lasst sich hierzu mittels des Bandantriebs 14 aus einer abgedichteten Vorratskammer 25 von einer darin befindlichen Abwickelspule 26 auf eine Aufwickelspule 28 umspulen, so dass die voneinander beabstandeten Testfelder 22 nacheinander an der Applikationsstelle 24 für sukzessive Tests zum Einsatz gebracht werden können. Hierbei wird nur die Aufwickelspule 22 durch den über einen Mitnehmerzapfen angekoppelten Bandantrieb 14 angetrieben. Denkbar ist auch der Einsatz von Stechelementen oder integrierten Stech/Testelementen als analytische Hilfsmittel auf einem Testband.

Der Bandantrieb 14 weist einen als hochdrehender Gleichstrom-Kleinmotor ausgebildeten Motor 30 und ein nachgeordnetes Untersetzungsgetriebe auf, das ein auf der Motorabtriebswelle 32 sitzendes Schneckenrad 34 und ein mehrstufiges Stirnradgetriebe 36 umfasst. Bei einer Kreisfrequenz des Motors 30 im Bereich von ca. 600 bis 1000 rad/s lässt sich damit die Spulendrehzahl der Aufwickelspule 28 auf weniger als 1 Umdrehung in der Sekunde reduzieren, so dass in energieeffizienter und kompakter Bauweise das erforderliche Drehmoment bereitsteht, um auch den Banddurchzug durch die Abdichtung der Vorratskammer 25 hindurch zu gewährleisten.

Mit der Regeleinrichtung 16 lassen sich die für DC-Kleinmotoren bauartbedingten hohen Drehzahlvarianzen kompensieren, um eine übermäßige Zugbelastung des Testbandes 20 zu vermeiden und eine vorgegebene Bereitstellungszeit eines unverbrauchten Testfelds 22 beispielsweise im Bereich von 5 bis 10 s zu gewährleisten. Hierbei ist zu berücksichtigen, dass die auf dem unverbrauchten Abschnitt des Testbandes 20 in der Vorratskammer 25 befindlichen Testfelder 22 erst auf Anforderung des Benutzers jeweils einzeln ausgespult werden, um schädliche Umwelteinflüsse auf die empfindliche Testchemie zu vermeiden.

Wie auch aus der Detailvergrößerung der Fig. 1 in axialer Draufsicht auf die Motorwelle 32 ersichtlich, umfasst die Regeleinrichtung 16 einen an dem Gleichstrommotor 30 angeordneten Drehgeber 38 zur Erfassung der IST-Drehzahl der Motorabtriebswelle 32. Dieser ist in dem gezeigten Ausführungsbeispiel als opto-elektronischer Encoder 40, speziell als auf der Motorabtriebswelle drehfest sitzendes Flügelrad 42 in Wirkverbindung mit einer das Flügelrad beidseitig umgreifenden, gerätefest angeordneten vierflügeligen Gabellichtschranke 44 ausgebildet.

Fig. 2 veranschaulicht den geschlossenen Regelkreis der Regeleinrichtung 16 in einem Blockschaltbild. Ein Vergleicher 46 ist zur Bildung einer Regeldifferenz e zwischen einer SOLL-Drehzahl $n_r$ bzw. SOLL-Kreisfrequenz $\omega_r$ (allgemein: $n = \omega/2\pi$) und der an der Motorwelle 32 mittels des Drehgebers 38 erfassten und über den Rückkopplungszweig 48 rückgespeisten IST-Kreisfrequenz $\omega$ ausgebildet. Ein eingangsseitig mit der Regeldifferenz e beaufschlagter Regelprozessor 48 sorgt für eine Reduzierung der Regeldifferenz. Zu diesem Zweck weist der Regelprozessor 48 ein Proportionalglied 50 und ein Integralglied 52 zweckmäßig in Form einer Software-Routine auf. Ausgangsseitig ist der Regelprozessor 48 mit einem Stellglied 56 gekoppelt, das als Pulsweitenmodulator den Gleichstrommotor 30 mit einer pulsweitenmodulierten Gleichspannung ansteuert. Der nachfolgende verzahnte Antriebsstrang 36 setzt die Antriebsrotation weitgehend abweichungsfrei in eine Translation des Testbandes 20 in der Bandkassette 18 um.

Fig. 3 veranschaulicht die genaue Bestimmung der IST-Drehzahl n aus dem Ausgangssignal 58 der Gabellichtschranke 44. Bei einer Umdrehung des vierflügeligen Flügelrads 42 in der Periodendauer T = 2n rad werden durch entsprechende Lichtschrankenunterbrechungen vier Impulse 60 erzeugt. Ein symbolisch dargestellter Taktgeber 62 zur Erzeugung von Zeittaktimpulsen 64 der Taktdauer d in Verbindung mit einem Digitalzähler 64 ermöglicht die Bestimmung der Impulszahl i zwischen zwei fallenden Flanken des Signals 58. Daraus ergibt sich die IST-Drehzahl n gemäß folgender Gleichung:

$$\omega \ (\mathrm{rad}\,/\mathrm{s}) = \frac{\frac{1}{4} * 2\pi}{i * d}$$

Bei dieser Methode wird die Drehzahlbestimmung mit zunehmender Winkelgeschwindigkeit ungenauer. Bei einer den Anforderungen entsprechenden maximalen Winkelgeschwindigkeit von $\omega$ = 1128 rad/s wird entsprechend der Zählerauslegung eine Impulszahl i = 1392 mit einer Zählertoleranz von $\pm1$ bestimmt. Daraus folgt eine minimale Genauigkeit der Drehzahlbestimmung von +0.07%, was für die gewünschte Genauigkeit der Drehzahlregelung hinreichend ist. Bei einer dem Fachmann bekannten Auslegung der Regelglieder 52, 54 lässt sich somit eine Bandgeschwindigkeit des Testbandes 20 von 15 +/- 2 mm/s mit einer Einstellzeit im Bereich von 0,1 bis 0,25 s erreichen.

Durch einen Sollwertgenerator 66 (Fig. 2) der Regeleinrichtung 16 wird die SOLL-Drehzahl nach Maßgabe einer gleichbleibenden Bereitstellungszeit für die jeweilige Bereitstellung der analytischen Hilfsmittel 22 festgelegt. Zweckmäßig wird hierzu der die Bandgeschwindigkeit bestimmende und mit zunehmendem Hilfsmittelverbrauch erhöhte Bandwickeldurchmesser auf der Aufwickelspule 28 durch die aktuelle Testnummer i der fortlaufend nummerierten Testfelder 22 berücksichtigt. Die aktuelle Testnummer i wird vorteilhafterweise in einem auf der Testbandkassette 18 angebrachten Speichermittel, beispielsweise einem RFID-Chip 68 (Fig. 1) gespeichert und in den Sollwertgenerator 66 eingespeist. Dieser enthält einen Sollwertspeicher 70, in welchem der Testnummer i für die auf dem Testband 20 verteilten fünfzig Testfelder jeweils ein Wert für die einzuregelnde Winkelgeschwindigkeit $\omega$ gemäß folgender Tabelle zugeordnet ist:

Tabelle I

| i | $\omega$ (rad/s) | i | $\omega$ (rad/s) | i | $\omega$ (rad/s) | i | $\omega$ (rad/s) | i | $\omega$ (rad/s) |
|---|---|---|---|---|---|---|---|---|---|
| 1 | 985 | 11 | 883 | 21 | 799 | 31 | 731 | 41 | 672 |
| 2 | 974 | 12 | 874 | 22 | 792 | 32 | 724 | 42 | 667 |
| 3 | 964 | 13 | 865 | 23 | 785 | 33 | 718 | 43 | 662 |
| 4 | 953 | 14 | 856 | 24 | 778 | 34 | 712 | 44 | 657 |
| 5 | 942 | 15 | 848 | 25 | 770 | 35 | 706 | 45 | 652 |
| 6 | 931 | 16 | 839 | 26 | 763 | 36 | 700 | 46 | 647 |
| 7 | 922 | 17 | 831 | 27 | 757 | 37 | 694 | 47 | 642 |
| 8 | 912 | 18 | 822 | 28 | 750 | 38 | 689 | 48 | 637 |
| 9 | 902 | 19 | 815 | 29 | 743 | 39 | 684 | 49 | 632 |
| 10 | 892 | 20 | 807 | 30 | 737 | 40 | 678 | 50 | 627 |

**Patentansprüche**

1. Analytisches Handgerät, insbesondere für Blutzuckertests, mit einer austauschbaren Testbandkassette (18), die ein mit einer Vielzahl von analytischen Hilfsmitteln (22) versehenes Testband (20) enthält, einem einen Gleichstrommotor (30) und ein mit der Testbandkassette (18) koppelbares Getriebe (34,36) aufweisenden Bandantrieb (14) zum sukzessiven Bereitstellen der analytischen Hilfsmittel (22), und einer Regeleinrichtung (16) zur Drehzahlregelung des Gleichstrommotors (30), **dadurch gekennzeichnet, dass** die Regeleinrichtung (16) einen an dem Gleichstrommotor (30) angeordneten Drehgeber (38) zur Erfassung der IST-Drehzahl der Motorabtriebswelle (32) aufweist.

2. Handgerät nach Anspruch 1, **dadurch gekennzeichnet, dass** der Drehgeber (38) einen die Drehung der Motorabtriebswelle (32) optisch abtastenden und dabei eine zur Drehzahl proportionale Rate von elektrischen Impulsen als Ausgangssignal erzeugenden opto-elektronischen Encoder (40) aufweist.

3. Handgerät nach Anspruch 1 oder 2, **dadurch gekennzeichnet, dass** der Drehgeber (38) einen drehfest auf der Motorabtriebswelle (32) sitzenden Unterbrecher (42), insbesondere ein Flügelrad oder eine Lochscheibe und eine mit dem Unterbrecher (42) zusammenwirkende gerätefeste Lichtschranke (44), vorzugsweise eine Gabellichtschranke aufweist.

4. Handgerät nach einem der Ansprüche 1 bis 3, **gekennzeichnet durch** einen zur Erzeugung von Zeittaktimpulsen ausgebildeten Taktgeber (62) und einen Zähler (64) zum Zählen von Zeittaktimpulsen zwischen zwei Signalflanken eines Ausgangssignals (58) des Drehgebers (38).

5. Handgerät nach einem der Ansprüche 1 bis 4,
**dadurch gekennzeichnet, dass** die Regeleinrichtung (16) einen Vergleicher (46) zur Bildung einer Regeldifferenz zwischen der IST-Drehzahl und einer als Führungsgröße vorgegebenen SOLL-Drehzahl aufweist.

6. Handgerät nach einem der Ansprüche 1 bis 5,
**dadurch gekennzeichnet, dass** die Regeleinrichtung (16) einen eingangsseitig mit einer Regeldifferenz beaufschlagbaren Regelprozessor (50) zur Einstellung einer Soll-Drehzahl in einem geschlossenen Regelkreis aufweist.

7. Handgerät nach einem der Ansprüche 1 bis 6,
**dadurch gekennzeichnet, dass** der Regelprozessor (50) vorzugsweise durch eine Software-Routine gebildete Proportional- und Integral-Regelglieder (52,54) aufweist.

8. Handgerät nach einem der Ansprüche 1 bis 7,
**dadurch gekennzeichnet, dass** die Regeleinrichtung (16) einen Pulsweitenmodular als Stellglied (56) zur Ansteuerung des Gleichstrommotors (30) mit einer pulsweitenmodulierten Gleichspannung aufweist.

9. Handgerät nach einem der Ansprüche 1 bis 8,
**dadurch gekennzeichnet, dass** die Regeleinrichtung (16) dazu eingerichtet ist, eine Bandgeschwindigkeit des Testbandes (20) von 15 +/- 2 mm/s mit einer Einstellzeit im Bereich von 0,1 bis 0,25 s zu erreichen.

10. Handgerät nach einem der Ansprüche 1 bis 9,
**dadurch gekennzeichnet, dass** die Regeleinrichtung (16) einen Sollwertgenerator (66) zur Festlegung einer aktuellen SOLL-Drehzahl nach Maßgabe einer gleichbleibenden Bereitstellungszeit für die jeweilige Bereitstellung der analytischen Hilfsmittel (22) aufweist.

11. Handgerät nach einem der Ansprüche 1 bis 10, **gekennzeichnet durch** einen Sollwertspeicher (70) zur Speicherung einer Sollwerttabelle, in welcher einer Testnummer der fortlaufend nummerierten analytischen Hilfsmittel jeweils ein Wert für die SOLL-Drehzahl des Gleichstrommotors (30) zugeordnet ist.

12. Handgerät nach einem der Ansprüche 1 bis 11,
**dadurch gekennzeichnet, dass** die Testbandkassette (18) mit einem insbesondere als RFID-Chip ausgebildeten Speichermittel (68) zur verbrauchsabhängigen Speicherung einer Testnummer des aktuell bereitzustellenden analytischen Hilfsmittels (22) versehen ist.

13. Handgerät nach einem der Ansprüche 1 bis 12,
**dadurch gekennzeichnet, dass** die analytischen Hilfsmittel (22) durch Bandtransport von einer gegenüber der Umgebung abgeschirmten Vorratsspule (26) abziehbar und an einer Applikationsstelle (24) bereitstellbar sind.

14. Verfahren zum Betrieb eines analytischen Handgeräts insbesondere nach einem der vorhergehenden Ansprüche, bei welchem ein mit einer Vielzahl von analytischen Hilfsmitteln (22) versehenes Testband (20) in Form einer austauschbaren Testbandkassette (18) in dem Handgerät (10) eingesetzt wird, wobei die analytischen Hilfsmittel (22) durch einen Bandantrieb (14) sukzessive bereitgestellt werden und dabei die Drehzahl eines Gleichstrommotors (30) des Bandantriebs (14) mittels einer Regeleinrichtung (16) geregelt wird, **dadurch gekennzeichnet, dass** die IST-Drehzahl der Motorabtriebswelle (32) durch einen an dem Gleichstrommotor (30) angeordneten Drehgeber (38) der Regeleinrichtung (16) erfasst wird.

Fig.1

Fig.2

Fig.3

EP 2 589 337 A1

| | Europäisches Patentamt European Patent Office Office européen des brevets | EUROPÄISCHER RECHERCHENBERICHT | Nummer der Anmeldung EP 11 18 7840 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | EP 2 177 155 A1 (HOFFMANN LA ROCHE [CH]; ROCHE DIAGNOSTICS GMBH [DE]) 21. April 2010 (2010-04-21) * Absatz [0018] - Absatz [0026] * * Abbildungen 1-3 * ----- | 1,6,9-14 | INV. A61B5/145 G01N33/487 |
| A | EP 2 279 689 A1 (HOFFMANN LA ROCHE [CH]; ROCHE DIAGNOSTICS GMBH [DE]) 2. Februar 2011 (2011-02-02) * Absatz [0011] * * Absatz [0014] * * Absatz [0018] - Absatz [0022] * * Abbildungen 1-4 * ----- | 1,13,14 | |
| A | US 2007/066938 A1 (IIO TOSHIAKI [JP] ET AL) 22. März 2007 (2007-03-22) * Absatz [0074] - Absatz [0078] * * Absatz [0086] - Absatz [0088] * * Absatz [0103] - Absatz [0116] * * Abbildungen 1,4 * ----- | 1-6,14 | |
| A | US 6 470 291 B1 (GOKER TURGUY [US] ET AL) 22. Oktober 2002 (2002-10-22) * Spalte 1, Zeile 12 - Zeile 15 * * Spalte 3, Zeile 39 - Spalte 4, Zeile 22 * * Spalte 6, Zeile 22 - Spalte 8, Zeile 16 * * Abbildung 1 * ----- | 1-6,14 | RECHERCHIERTE SACHGEBIETE (IPC) G01N A61B |

Der vorliegende Recherchenbericht wurde für alle Patentansprüche erstellt

| Recherchenort Berlin | Abschlußdatum der Recherche 28. März 2012 | Prüfer Völlinger, Martin |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
........................................................
& : Mitglied der gleichen Patentfamilie, übereinstimmendes Dokument

EPO FORM 1503 03.82 (P04C03)

**EP 2 589 337 A1**

**ANHANG ZUM EUROPÄISCHEN RECHERCHENBERICHT**
**ÜBER DIE EUROPÄISCHE PATENTANMELDUNG NR.**

EP 11 18 7840

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

28-03-2012

| Im Recherchenbericht angeführtes Patentdokument | Datum der Veröffentlichung | Mitglied(er) der Patentfamilie | Datum der Veröffentlichung |
|---|---|---|---|
| EP 2177155 A1 | 21-04-2010 | AU 2009306499 A1<br>CA 2747295 A1<br>CN 102186410 A<br>EP 2177155 A1<br>EP 2365775 A1<br>KR 20110074873 A<br>TW 201037310 A<br>US 2011236276 A1<br>WO 2010046323 A1 | 29-04-2010<br>29-04-2010<br>14-09-2011<br>21-04-2010<br>21-09-2011<br>04-07-2011<br>16-10-2010<br>29-09-2011<br>29-04-2010 |
| EP 2279689 A1 | 02-02-2011 | EP 2279689 A1<br>TW 201121507 A<br>WO 2011012645 A1 | 02-02-2011<br>01-07-2011<br>03-02-2011 |
| US 2007066938 A1 | 22-03-2007 | KEINE | |
| US 6470291 B1 | 22-10-2002 | KEINE | |

EPO FORM P0461

Für nähere Einzelheiten zu diesem Anhang : siehe Amtsblatt des Europäischen Patentamts, Nr.12/82

8